(19) Europäisches Patentamt
European Patent Office
Office européen des brevets

(11) **EP 1 528 936 B1**

(12) **EUROPEAN PATENT SPECIFICATION**

(45) Date of publication and mention of the grant of the patent:
**12.10.2016 Bulletin 2016/41**

(21) Application number: **03787765.1**

(22) Date of filing: **12.08.2003**

(51) Int Cl.:
*A61K 39/395* (2006.01)    *A61K 39/00* (2006.01)
*G01N 33/53* (2006.01)    *A61P 35/00* (2006.01)

(86) International application number:
**PCT/EP2003/008933**

(87) International publication number:
**WO 2004/016285 (26.02.2004 Gazette 2004/09)**

(54) **COMBINATION OF TUMOR-ASSOCIATED SURFACE PROTEIN ANTIGENS AND TUMOR-ASSOCIATED SUGARS IN THE TREATMENT AND DIAGNOSIS OF CANCER**

KOMBINATION VON TUMOR-ASSOZIIERTEN OBERFLÄCHENANTIGENEN MIT TUMOR-ASSOZIIERTEN ZUCKERSTOFFEN FÜR DIE BEHANDLUNG UND DIAGNOSE VON KREBS

COMBINAISON D'ANTIGENES D'UNE PROTEINE DE SURFACE ASSOCIES A UNE TUMEUR ET DE SUCRES ASSOCIES A UNE TUMEUR DANS LE TRAITEMENT ET LE DIAGNOSTIC DU CANCER

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **12.08.2002 AT 12162002**

(43) Date of publication of application:
**11.05.2005 Bulletin 2005/19**

(73) Proprietors:
• **greenovation Biotech GmbH**
  **79111 Freiburg (DE)**
• **Meridian Biopharmaceuticals GmbH**
  **1230 Wien (AT)**

(72) Inventors:
• **LOIBNER, Hans**
  **1238 Wien (AT)**
• **HIMMLER, Gottfried**
  **1180 Wien (AT)**

(74) Representative: **Sonn & Partner Patentanwälte**
**Riemergasse 14**
**1010 Wien (AT)**

(56) References cited:
**WO-A-00/06605**    **WO-A-01/35989**
**WO-A2-03/003985**

• **FLIEGER DIMITRI ET AL: "A bispecific single-chain antibody directed against EpCAM/CD3 in combination with the cytokines interferon alpha and interleukin-2 efficiently retargets T and CD3+CD56+ natural-killer-like T lymphocytes to EpCAM-expressing tumor cells" CANCER IMMUNOLOGY IMMUNOTHERAPY, vol. 49, no. 8, October 2000 (2000-10), pages 441-448, XP002263709 ISSN: 0340-7004**
• **KIM^1 S K ET AL: "Effect of immunological adjuvant combinations on the antibody and T-cell response to vaccination with MUC1-KLH and GD3-KLH conjugates" VACCINE, BUTTERWORTH SCIENTIFIC. GUILDFORD, GB, vol. 19, no. 4-5, 15 October 2000 (2000-10-15), pages 530-537, XP004218125 ISSN: 0264-410X**
• **DI CARLO ANGELINA ET AL: "Evaluation of epidermal growth factor receptor, carcinoembryonic antigen and Lewis carbohydrate antigens in human colorectal and liver neoplasias" ONCOLOGY REPORTS, vol. 8, no. 2, March 2001 (2001-03), pages 387-392, XP009022189 ISSN: 1021-335X**
• **GRATSA ANASTASIA ET AL: "Correlation of expression of NCAM and GD3 ganglioside to motile behavior in neoplastic glia" ANTICANCER RESEARCH, vol. 17, no. 6B, November 1997 (1997-11), pages 4111-4117, XP009022147 ISSN: 0250-7005**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- NANASHIMA A ET AL: "High serum concentrations of sialyl Tn antigen in carcinomas of the biliary tract and pancreas." JOURNAL OF HEPATO-BILIARY-PANCREATIC SURGERY. JAPAN 1999, vol. 6, no. 4, 1999, pages 391-395, XP002263710 ISSN: 0944-1166
- SAUTER GUIDO ET AL: "Lewis Y/EpCAM co-expression in breast cancer is correlated with poor prognosis." PROCEEDINGS OF THE AMERICAN ASSOCIATION FOR CANCER RESEARCH ANNUAL, vol. 44, July 2003 (2003-07), page 35 XP001156672 94th Annual Meeting of the American Association for Cancer Research;Washington, DC, USA; July 11-14, 2003, July 2003 ISSN: 0197-016X
- SANDERS D S A ET AL: "Lewis blood group and CEA related antigens;coexpressed cell-cell adhesion molecules with roles in the biological progression and dissemination of tumours" MOLECULAR PATHOLOGY, vol. 52, no. 4, August 1999 (1999-08), pages 174-178, XP009022199 ISSN: 1366-8714
- LIVINGSTON ET AL: 'IMPROVED SURVIVAL IN STAGE III MELANOMA PATIENTS WITH GM2 ANTIBODIES: A RANDOMIZED TRIAL OF ADJUVANT VACCINATION WITH GM2 GANGLIOSIDE' JOURNAL OF CLINICAL ONCOLOGY vol. 12, no. 5, 1994, pages 1036 - 1044
- KIM ET AL: 'COMPARISON OF THE EFFECT OF DIFFERENT IMMUNOLOGICAL ADJUVANTS ON THE ANTIBODY AND T-CELL RESPONSE TO IMMUNIZATION WITH MUC1-KLH AND GD3-KLH CONJUGATE CANCER VACCINES' VACCINE vol. 18, no. 7/8, 2000, pages 597 - 603
- RAGUPAHTI ET AL: PROCEEDINGS OF THE ANNUAL MEETING OF THE AMERICAN ASSOCIATION FOR CANCER RESEARCH, vol. 41, no. 874/875, 2000, page 5557,
- ZHANG S ET AL: "Expression of potential target antigens for immunotherapy on primary and metastatic prostate cancers.", CLINICAL CANCER RESEARCH : AN OFFICIAL JOURNAL OF THE AMERICAN ASSOCIATION FOR CANCER RESEARCH FEB 1998 LNKD- PUBMED:9516914, vol. 4, no. 2, February 1998 (1998-02), pages 295-302, ISSN: 1078-0432
- KLINGER M ET AL: "INHIBITION OF SIGNALING VIA ERBB-RECEPTORS (EGFR AND HER2/NEU) BY ANTIBODIES THAT TARGET THE LEWIS Y-ANTIGEN", AMERICAN ASSOCIATION FOR CANCER RESEARCH. PROCEEDINGS OF THE ANNUAL MEETING, AMERICAN ASSOCIATION FOR CANCER RESEARCH, US, vol. 44, 1 July 2003 (2003-07-01), page 1359, XP008024266, ISSN: 0197-016X
- AZUMA A ET AL: "AUGMENTED LUNG ADENOCARCINOMA CYTOTOXICITY BY THE COMBINATION OF A GENETICALLY MODIFIED ANTI-LEWIS Y ANTIBODY AND ANTIBODIES TO COMPLEMENT REGULATORY PROTEINS", SCANDINAVIAN JOURNAL OF IMMUNOLOGY, BLACKWELL SCIENCE PUBL., OXFORD, GB, vol. 42, no. 2, 1 January 1995 (1995-01-01), pages 202-208, XP000941182, ISSN: 0300-9475
- RAMSLAND P A ET AL: 'Structural Convergence of Antibody Binding of Carbohydrate Determinants in Lewis Y Tumor Antigens' JOURNAL OF MOLECULAR BIOLOGY vol. 340, no. 4, 16 July 2004, ACADEMIC PRESS, UNITED KINGDOM, pages 809 - 818, XP004516692 DOI: 10.1016/J.JMB.2004.05.037 ISSN: 0022-2836

Remarks:

The file contains technical information submitted after the application was filed and not included in this specification

**Description**

The invention relates to a set for combined application for the treatment of cancer patients, as well as its diagnostic use. Also, a method of screening of neoepitopes and their use is disclosed.

Cancer is a wide-spread disease and is lethal in many cases. The therapy of cancer usually comprises the removal of a solid tumor, and a further treatment which is to prevent and reduce, respectively, metastases. Besides surgery, the standard therapies include chemotherapy and radiation therapy. Despite the comprehensive therapy which often involves severe side effects, the success of treatment is insufficient. Most frequently, cancer forms with epithelial tumors occur, which inter alia concern breast, stomach intestines, pancreas, lungs, prostate and ovaries. The relapse rate in intestinal cancer is approximately 45%. Metastatic epithelial cancer is considered to be nearly incurable. Therefore, in the treatment of cancer patients it is important to prevent, and reduce, respectively the formation of metastases.

Tumor cells are capable of disseminating from primary tumors in body liquids and other organs. These disseminated tumor cells may be in their dormant state and often cannot be attacked by a chemotherapy (radiotherapy). Such a treated patient seems to be in a cured state, which is described as "minimal residual disease". Dormant tumor cells, however, have a potential of forming metastases if they become growing and metastasizing cells.

Immunotherapy constitutes an innovative possible treatment of cancer patients. Both active and also passive immunotherapy are acknowledged measures for supporting the immune system.

The adaptive immune system of humans consists of two essential components, the humoral and the cellular immunity. The adaptive immune response partially is based on the clonal selection of B- and T-lymphocytes and in principle allows for the recognition of any desired antigen as well as for the build-up of an immunological memory. These characteristics of the adaptive immune system are generally usefully addressed in vaccinations.

Each B-cell produces an antibody with a defined binding specificity. This antibody is also present as a specific receptor in the membrane of the B-cell producing it. The humoral immune response against antigens recognized as foreign is based on the selective activation of those B-cells which produce such antibodies that can bind to an epitope of the respective antigen. For the antibody diversity, DNA rearrangements in the course of B-cell differentiation play a decisive role.

There are several possible ways of interfering in the immune system. One approach of relatively specifically destroying tumor cells is the passive immunotherapy with antibodies directed against tumor-associated antigens (TAA) (Immunology Today (2000), 21:403-410; Curr. Opin. Immunol. (1997), 9:717). Another approach of destroying tumor cells is the active vaccination which triggers an immune response against TAA. This immune response thus is also directed against the corresponding tumor cells (Ann. Med. (1999), 31:66; Immunobiol. (1999), 201:1).

1. Passive antibody therapy:

For therapeutic purposes, it is possible to supply to an organism antibodies required for a certain function within this organism. This type of application is called passive immunotherapy, and it can be used in various medical indications, e.g. in the immunotherapy of cancer (Immunol. Today (2000), 21:403), intoxications (Toxicon (1998), 36:823; Therapie (1994), 49:41) and infections (Clin. Infect. Dis. (1995), 21:150). In these cases, antibodies can be used which either have been derived from appropriately immunized animals or can be recovered from cells by various biological or molecular-biological techniques (e.g. hybridoma technique, phage-display technique, etc.) via the immortalization of immunoglobulin genes.

2. Active immunization:

To modulate the immune system, an immunization with antigens can be used. Antigens are molecules, molecule complexes or whole organisms to which antibodies can bind. Not all the antigens induce an immune response, i.e. not all the antigens are immunogenic. Certain small molecules are not registered by the immune system (haptens); such smaller molecules can be presented to the immune system in suitable form, and thus be made immunogenic. Such a method is the coupling of the hapten to an immunogenic molecule, a so-called carrier molecule. For an active immunization, also antibody preparations can be used, as described in EP 1140168.

Tumor cells can be attacked by the immune system only to a limited extent, since they are hardly different from normal cells and specific antibodies therefore are missing. Much research is directed to the identification of suitable targets, i.e. target antigens, for the preparation of tumor-specific antibodies. The immunotherapy for the treatment of cancer then either comprises the passive therapy by the direct administration of the specific antibodies, or the active vaccination with suitable antigen-targets for stimulating the immune system and generating the specific antibodies in vivo.

Certain TAAs are defined as relevant "targets" for the development of immunotherapeutic agents for the prophylaxis and/or treatment of cancer. TAAs are structures which preferably are expressed on the cell membrane of tumor cells, thereby allow for a differentiation relative to non-malignant tissue, and thus can be viewed as targets for the diagnostic and therapeutic applications of specific antibodies.

Beside other physiological characteristics which distinguish them from normal cells, cancer cells practically always have a changed type of glycosylation (Glycoconj. J. (1997), 14:569; Adv. Cancer Res. (1989), 52:257; Cancer Res. (1996), 56:5309). Even though the changes are different from tissue to tissue, it can be found that an aberrant glycosylation is typical of cancer cells. In most instances, the changed glycosylation is presented at the surface of cells in the form of glycoproteins and glycolipids. These changed sugar structures therefore can be designated as TAAs which in many cases are sufficiently tumor-specific, i.e. they rarely appear in "normal cells". In many instances, the cells do not have a uniform glycosylation, and neither do the tumor cells, i.e. there exist various glyco-forms of complex glycan chains on a cell (Annu. Rev. Biochem. (1988), 57:785).

In the course of the discovery and the subsequent characterization of various TAAs it has been found that they have important functions for cancer cells. They allow the degenerate cells to have properties characteristic of the malignant phenotype, such as, e.g., an increased adhesion capacity, or an increased uptake of growth factors, which are highly important for establishing metastases. However, in certain stages, such antigens may very well also be expressed on normal cells where they are responsible for normal functions of these cells. An example of this is the Lewis Y carbohydrate antigen which appears on the plurality of tumors of epithelial origin, but also plays an important role during the fetal development of epithelial tissues. It has been shown that the expression of this antigen in lung cancer is associated with an unfavorable prognosis, since Lewis Y positive cancer cells apparently have a higher metastatic potential (N. Engl. J. Med. 327 (1992), 14).

In EP 0 528 767, the use of a humanized anti-Lewis Y antibody for the treatment of epithelial cancer has been described.

Among the further known tumor-associated carbohydrate structures, there are, e.g., all those Lewis antigens which are increasingly expressed in many types of epithelial cancers. Among them are Lewis x- and Lewis b-structures in addition to Lewis y-structures, as well as sialylated Lewis x-structures. Other carbohydrate antigens are GloboH-structures, KH1, Tn antigen, sialylTn, TF antigen, the alpha-1,3-galactosyl epitope (Elektrophoresis (1999), 20:362; Curr. Pharmaceutical Design (2000), 6:485, Neoplasma (1996), 43:285).

Other TAAs are proteins which are particularly highly expressed by cancer cells, such as, e.g. CEA, TAG-72, MUC1, Folate Binding Protein A-33, CA125, EpCAM, HER-2/neu, PSA, MART, etc. (Sem. Cancer Biol. (1995), 6:321). Relevant TAAs often are surface antigens of epithelial cells which occur in larger numbers in growing cells, such as fetal tissue, and also in tumor tissue. A special group of TAAs are involved in the adhesion processes of the epithelial cells. Among the cellular adhesion proteins which are over-expressed on tumor cells are EpCAM, NCAM and CEA.

In Austrian application A 744/2002, an immunogenic antibody having at least two different epitopes of a TAA has been described. A preferred antibody comprises at least one epitope of EpCAM and one epitope of Lewis Y.

According to Allergol. et Immunopathol. 25, 4 (176-81), 1997, the MUC-1 gene product, the polymorphous epithelial mucine, and the development of specific monoclonal antibodies is described. As the target for the TAA mucine, both the peptide sequence and also specific carbohydrates are examined.

Direct therapeutical applications of antibodies against TAA are based on passive immunotherapies, i.e., a specific antibody is systemically administered in a suitable amount to cancer patients, and has an immunotherapeutic effect. The biological half-life of such agents will depend on their structure and is limited. Therefore, it is necessary to carry out repeated applications. When using xenogenic antibodies (e.g. monoclonal antibodies, MABs, for example of murine origin) however, this can lead to undesired immune reactions which may neutralise a possible therapeutic effect and may cause dangerous side effects (eg. immune complex formation with kidney failure, anaphylactic reactions). Therefore, such immunotherapeutic agents can be administered for a limited time only.

A better tolerance is obtained by reducing the xenogenic structures of the antibody and by introducing human structures, e.g. with chimeric or humanized antibodies. Also systems for producing specific human antibodies are developed. Thus, according to the prior art, certain cell lines, organisms or transgenic animals can produce human monoclonal antibodies.

Flieger D. et al. (2000), Cancer Immunology Immunotherapy 49:441-448, describes a method to enhance the cytotoxicity of "natural-killer-like" T-lymphocytes against EpCAM expressing tumor cells.

Kim S.K. et al. (2000), Vaccine 19:530-537 relates to the use of adjuvants to enhance the immune response of vaccines directed against two cancer antigens (GD3 ganglioside and MUC1 peptide) covalently bound to keyhole limpet hemocyanin.

WO 01/35989 A discloses the purification of anti-idiotypic antibodies using resin bound specific antibodies directed against EpCAM and Lewis y for the production of autologous vaccines.

WO 00/06605 A discloses the recombinant production of multifunctional antibody derivatives ("heterominibodies") and their use in the treatment of cancer.

According to Di Carlo A. et al. (2001), Oncology Reports 8:387-392, different tumor tissues were analysed in order to identify specific markers.

Gratsa A. et al. (1997), Anticancer Research 17:4111-4117 discloses a study wherein the connection of the expression of tumor markers (NCAM and gangliosides) and the mobility of tumor cells in the nervous system was analysed.

In Nanashima A. et al. (1999), Journal of Hepato-Biliary-Pancreatic Surgery 6:391-395 the determination of SialylTn antigen in serum of cancer patients is described without determing SialylTn antigen or other markers on tumor cells or of solid tumors.

Sanders D.S.A. et al. (1999), Molecular Pathology 52:174-178 gives a general overview of Lewis and CEA antigens and their role in the interaction of cells of different tissues.

Livingston et al., Journal of Clinical Oncology 12(5) (1994): 1036-1044 describes the vaccination of melanoma patients with GM2-ganglioside and bacille Calmette-Guerin.

WO 03/003985 A describes multivalent vaccines with conjugated antigens.

Kim et al., Vaccine 18 (2000): 597-603 describes MUC-1-KLH-conjugate and GD3-KLH-conjugate vaccines.

Ragupahti et al., Proceedings of the annual meeting of the American Association for Cancer Research 41(874/875) (2000): 5557, describes polyvalent antigen conjugate vaccines.

Zhang et al., Clin. Can. Res 1998, 4(2), 295-302, relates to a screening of certain cancerous and non-cancerous tissues for the presence of various antigens, including Lewis Y and KSA.

The invention has as its object to improve the immunotherapeutic treatment of cancer patients by the selection of suitable target antigens and the accompanying diagnostics

According to the invention, this object is achieved by the subject matter of the claims. The following description describes the invention where related to the claims.

According to the invention, a kit is provided which is defined as follows :

A kit for the combined use for the treatment of cancer patients, which set comprises the following components:

a) an antibody directed against a tumor cell surface protein, which is Lewis Y glycosylated in cancer, selected from a receptor of the EGF-receptor family and EpCAM, and

b) an antibody directed against the Lewis Y antigen, said antibodies a) and b) having antibody-dependent cellular cytotoxicity and bind to the tumour cells in the patient thereby forming immune complexes.

Besides other physiological characteristics which distinguish them from normal cells of the respective tissue, cancer cells practically always have a changed type of glycosylation as has been mentioned before (Glycoconj. J. (1997), 14:569; Adv. Cancer Res. (1989), 52:257; Cancer Res. (1996), 56:5309). Even though the changes are different from tissue to tissue, it can be found that an aberrant glycosylation is typical of cancer cells (as compared to the corresponding normal cells in this tissue). In most instances, the changed glycosylation will be presented at the surface of the cells in the form of glycoproteins and glycolipids. These changed sugar structures therefore can be designated as TAAs, which in many instances are sufficiently tumor-specific, i.e. they rarely occur in "normal" cells. In many instances, the cells, and also the tumor cells, do not produce a uniform glycosylation, i.e. there exist various glycoforms of complex glycan chains on one cell (Annu. Rev. Biochem. (1988), 57:785).

The combination of the active substances relates to an antibody-antibody combination. With the inventive kit, effective immunotherapies directed against tumor-specific target antigens are rendered possible.

Surprisingly, it has been shown that the common, simultaneous, parallel or consecutive specific formation of the immune complexes of antibodies with the antigens of the components of the kit contributes particularly to the positive course of the disease in a cancer patient. In particular, it has been found that cancer patients who have both target antigens together on solid tumors have an unproportionately worse prognoses of the survival time as compared to patients with only one of the target antigens. The immunotherapeutic treatment with the inventive combination of the tumor-associated components therefore is considered as particularly effective for increasing the survival time or the relapse-free time, respectively.

The target antigens of the component a) preferably constitute at least one epitope of a cellular adhesion protein, and preferably they are selected from the homophilic adhesion proteins of epithelial tumor cells. They have the properties that they can bind to the same surface proteins of other tumor cells and thus are capable of forming a cell agglomerate. Among them are antigens which are particularly derived from the EpCAM antigen, the EpCAM molecule itself or epitopes, parts or mimics thereof. A particularly good immunogen for an active immunization with an antigen of component a) is an immunogenic antibody as described in EP 1140168 B1. Further antigens of cellular adhesion proteins have been derived either from NCAM or from CEA.

According to a further preferred embodiment, the antigen of component a) has at least one epitope of a surface receptor, in particular a receptor molecule selected from the group of the EGF receptor family, among them the EGF receptor and Her-2/neu receptor. Yet, moreover, also antigens with epitopes of a mucine, in particular MUC1, or CA125, can be used.

Among the epitopes relevant for component a), preferably there is at least one epitope of a human antigen selected from the group of peptides or proteins with regulating function for cellular adhesion processes and receptor functions. Besides the already mentioned antigens, they particularly include also T-cell peptides which preferably have been derived from the TAAs.

An antigen of component b) of the inventive kit is directed to the immunotherapy against an epitope of a carbohydrate. Carbohydrate epitopes preferred according to the invention are tumor-associated, aberrant carbohydrate structures, such as the Lewis antigens, e.g. Lewis x-, Lewis b- and Lewis y-structures, as well as sialylated Lewis x-structures. Moreover, also GloboH structures, KH1, Tn antigen, TF antigen, the alpha-1,3-galactosyl-epitope are carbohydrate antigen structures .

A particularly good target for component b) according to the invention is the Lewis Y antigen. Particularly preferred is the use of a humanized antibody, such as described in EP 0 528 767. This antibody recognizes Lewis Y antigen on tumor cells or on surface receptors of tumor cells, respectively, and is suitable for the passive immunotherapy.

It has been found that the surface receptors of a tumor cell are equipped with an aberrant glycosylation and form relevant epitopes as defined by the invention. This primarily relates to the EGF receptor family, among them the EGF receptor or Her-2/neu receptor. Additionally, also neoepitopes being formed by the aberrant glycosylation of an antigen of a cellular surface protein with a carbohydrate structure are described.

By the immunotherapy with the target of the aberrant glycosylation, practically all tumor-specific receptors which are characterized by this aberrant glycosylation are blocked. Among them are, e.g., all the receptors of the EGF-receptor family, the CD55 (791Tgp72/DAF - decay accelerating factor) receptor, the transferrin receptor, and the P-glycoprotein.

It has also been found that antibodies which are directed against an aberrant glycosylation bind in a functional manner to several receptors of the family of the EGF receptors and thus the signal cascade for inducing the cell growth can effectively be blocked. In Austrian application A 995/2002 it could be demonstrated that it was possible to functionally bind in particular the erk1 and erk2 isoforms of the MAP kinase by means of the antibodies. The binding of the growth factors to the receptors was thereby prevented or reduced, respectively. This treatment is more specific as compared to immunotherapy using antibodies against the proteinaceous extracellular part of the EGF receptor, since the unusual tumor-associated carbohydrate structures are missing on the EGF receptors of normal cells. On the other hand, the treatment is more universal, since simultaneously different receptors having the same aberrant glycosylation are blocked.

By the use of the immunotherapy, directed against an aberrant glycosylation, thus also the mitogenic stimulation of a cancer cell by EGF or heregulin is prevented. The specific binding of the antibodies to a tumor-associated glycosylation of cancer cells blocks the interaction of the receptors of growth factors with their physiologic ligands and inhibits the signal transduction through these receptors and thus, the cell growth.

At the same time, such an antibody can specifically attack the tumor cell by its effect within the humoral and cellular immune system. Tumor cells which express the EGF receptor or receptors of the EGF receptor family, respectively, according to the invention are specifically bound and can be lysed or be blocked in growth.

The inventive combination of the target antigens relates to an epitope of the EpCAM molecule or of the Her-2/neu receptor for the component a), and an epitope of the Lewis Y molecule for the component b).

The target antigens are either directly affected by the specific immunotherapy, or indirectly, by the binding of TAA which substantially negatively affect a function of the target antigens. Target antigens particularly comprise epitopes of proteinaceous antigens, glycoproteins or carbohydrate antigens. As a rule, it can be assumed that by a proteinaceous antigen a polypeptide of at least five amino acids is to be understood.

Preferred epitopes are derived from antigens which are specific of epithelial tumors and increasingly occur e.g., in breast cancer, cancer of the stomach and intestines, such as colon and rectal, the prostate, pancreas, ovaries and the lungs. Among the preferred epitopes are those which primarily induce a humoral immune response, i.e. a specific antibody formation, in vivo. On the other hand, also those antigens can be chosen as epitopes as defined by the invention which generate a T-cell specific immune response. Among them also intracellular structures or T-cell peptides can be found. Suitable epitopes are expressed at least in 20%, preferably at least in 30% of the cases by tumor cells of a certain type of cancer, more preferred in at least 40%, in particular in at least 50% of the patients.

Methods of finding suitable antigen structures, modelling and producing the TAA-derived peptides, polypeptides or proteins, or the nucleic acids coding therefor, furthermore, lipoproteins, glycolipids, carbohydrates or lipids are known to the person skilled in the art and can be provided for the respective tumor-specific structure without undue experimental expenditures. Methods for conjugating or derivatizing such structures which likewise are suitable according to the invention are also known. Furthermore, the methods of producing the specific antibodies that are suitable according to the invention are known.

For selecting suitable antigenic structures, or corresponding antibodies or immune complexes, respectively, a suitable antigen and/or a corresponding antibody is selected by an immunological screening method. The method for the immunological selection of a tumor-specific target antigen or of antibodies directed against the target antigen employs a diagnostic agent. According to the invention, this agent comprises the active substances of the afore-mentioned components a) and b) which both form immune complexes with an immunotherapeutic candidate either simultaneously or independently of each other.

Screening may be carried out with the assistance of known methods, among them phage display methods and hybridoma technology, and the immunoreagents corresponding to components a) and b), or also with qualitative or preparative methods for the selective binding of the candidate antigens to the immune reagents. An appropriate screening for the first time also allows for the selection and the preparation of a neoepitope which is just formed by the glycosylation of an antigen of component a) with a carbohydrate structure of component b). It has been found that this neoepitope is then expressed by tumor cells if a cancer patient has an unproportionately unfavorable prognosis. According to the invention, it has now become possible for the first time to find the tumor-specific and relevant neoepitopes with the assistance of a screening method, and to develop appropriate immunotherapies. After the neoepitope has been characterized, suitable antibody preparations can be prepared which will recognize just this neoepitope, and preferably will not recognize one or both of the individual antigens of components a) and b). The immunotherapy with the target of the neoepitope has been improved insofar as epithelial cells of normal tissue will not be affected, but merely the tumor cells.

Examples of such neoepitopes are epitopes which are formed by the glycosylation of an EpCAM protein or a Her-2/neu receptor with Lewis Y carbohydrate or appropriately sialylated glycoproteins. When antibodies with a specificity for these neoepitopes are produced and prepared, they preferably do not bind to the de-glycosylated proteins nor do they bind to the carbohydrate motif on structurally different proteins. It is precisely these antibodies which preferably are suggested as monoclonal antibodies for the passive immunotherapy so as to avoid unspecific interactions and side effects. The identification of the neoepitopes can also be the basis for the development of vaccination antigens, by presenting an immunogen with exactly this epitope. This epitope or a mimic of the epitope can be produced easily from appropriate peptide libraries or by anti-idiotypic antibody techniques or also as a derivative, e.g. a fragment, of a naturally occurring antigen. On the basis of the selected neoepitope, a preparation of an antigen is obtainable which has exactly this neoepitope or the mimic thereof, eg an anti-idiotypic antibody, mimotope. Such antigen preparations are valuable active substances for the active immunization of cancer patients or they can also be employed as a diagnostic preparation.

A tumor cell line which expresses a Lewis Y glycosylated Her-2/neu receptor is used for producing the respective antibodies. With a selection method that uses the individual immunoreagents Lewis Y, Her-2/neu and a Lewis Y glycosylated. Alternatively, Lewis Y can be synthetically produced. Her-2/neu those antibodies are recovered which merely bind to the combination antigen, yet not to one of the separate antigens. These antibodies can be the basis for the development of the neoepitope-specific mimics.

Hybridomas can be recovered from immunized mice, the selection of the positive clones being effected by a differential screening with the components of the inventive kit. It will then be possible to identify a neoepitope by a usual epitope mapping method in that the specific antibodies bind to the glycosylated surface protein, yet not to the individual components a) or b). From a positive clone, an anti-neoepitope monoclonal antibody can then be obtained. A preparation of such an antibody or a derivative thereof is, e.g., suitable for the passive immunotherapy or for diagnostic use.

In a particular embodiment, for at least one of the components a) and b) an antibody mixture is provided of different antibodies with a specificity for one or more of the antigens from a) or b). In particular, it is possible to provide antibody mixtures as a representative panel having specificity for at least two equal or different epitopes of an adhesion protein, such as EpCAM, or an aberrant glycosylation, e.g. of the Lewis carbohydrate antigens.

The immunotherapy is carried out with a pharmaceutical preparation which comprises either both components, i.e. antigens and/or antibodies of the kit together or as separate preparations, in pharmaceutically acceptable form. A kit according to the invention thus preferably comprises the components a) and b) in one preparation each or in one single pharmaceutical preparation which is suitable for immunotherapy. Alternatively, when using the neoepitope according to the invention, only one component might be useable.

The kit can be used both for an active immunotherapy and also for a passive immunotherapy, or for the combination of the active and passive immunotherapy, respectively.

A medicament used according to the invention for the passive immunotherapy preferably is provided in a suitable formulation. Preferred are such formulations with a pharmaceutically acceptable carrier. The latter comprises, e.g., auxiliary substances, buffers, salts and preserving agents. Preferably, a ready-to-use infusion solution is provided.

Since an antibody is comparatively stable, medicaments based on antibodies or their derivatives have the substantial advantage that they can be put on the market as storage-stable solutions or as a formulation in a ready-to-use form. The latter is preferably storage-stable at refrigerating temperature up to room temperature.

The medicament formulated for intravenous administration may, however, also be stored in frozen or lyophilized form and may be thawed or reconstituted, respectively, upon demand.

For the passive immunotherapy, and thus for binding the relevant surface antigens to tumor cells, usually a high dose of at least 50 mg, preferably at least 100 mg, most preferred at least 200 mg, is administered per patient. The maximum dose is limited by the tolerability of the antibody and will depend on its specificity and avidity. Humanized antibodies and human antibodies, respectively, are the best tolerable. A dose of up to 1 g or in some cases of up to 2 g per patient and treatment may very well be advantageous.

The usual treatment for the passive immunotherapy comprises repeated infusions at regular intervals, e.g. weekly, for a period of time of from 6 to 24 weeks, at a dose ranging from 1 to 10 mg/kg, preferably ranging from 2 to 6 mg/kg. An antibody preparation can also be administered locally, i.e. to the tumor tissue and/or intraoperatively into the wound region after removal of a tumor. Various modes of administration may appropriately be combined, e.g. an i.v. infusion shortly before surgery, as well as a local dose directly into the wound region during surgery. Practical application means for the localized administration comprise, e.g., ready-to-use applicating means, such as catheters, syringes or sprays, suitable for distributing liquid medicaments.

The i.v. treatment is preferably repeated at certain time intervals, corresponding to the half-life of the antibody used which usually is in the range of from 5 to 30 days. By a special derivatization, eg. pegylation of the antibody it is possible to lengthen the half-life to up to several months, and to thereby lengthen the treatment intervals accordingly.

The concentration of the active substance of the medicament will depend on its tolerability. A particularly well tolerated preparation based on a humanized antibody can be administered directly to the patient at a high concentration and without being further diluted. By the preferred concentration in the range of from 0.1% to 10%, preferably 1% to 5%, it is possible to keep low the administered volume and the respective infusion time. An antibody solution used according to the invention can be administered intravenously as a bolus injection of a concentrated solution, or also in diluted form. The medicament can be diluted e.g. 1:10 to 1:100-fold with physiological saline solution so as to provide an infusion preparation.

By the term "antibody" antibodies of all types are to be understood, in particular monospecific or polyspecific monoclonal antibodies, or also chemically, biochemically or molecular-biologically prepared antibodies, or polyclonal antibodies having a certain specificity, e.g. an immune serum or a fraction of an immune serum.

An antibody utilized according to the invention preferably is a native, i.e. functionally active, antibody. This antibody preferably does not have an attached label or other detection agent so as not to impair its functionality. Native antibodies have the properties of the antibodies naturally occurring in patients. Native antibodies are heterotetrameric glycoproteins composed of two identical light chains and two identical heavy chains.

An antibody used according to the invention preferably is of the type of an immunoglobulin, such as an IgG, IgE, IgM, IgA or IgD.

According to the invention, the antibody binds directly to a tumor cell or to metastases, or micrometastases, respectively. A thereby formed immune complex of the antibody is the prerequisite for the humoral and cellular activities of the immune system, expressed by an antibody-dependent cellular cytotoxicity (ADCC) and/or a complement dependent cytotoxicity (CDC) effector function. These effector functions are determined by standard tests for the detection of function-blocking, receptor-blocking and prevention of adhesion.

High-affinity antibodies are preferred according to the invention. In particular, antibodies are used which bind with an affinity corresponding to a dissociation constant of below a Kd value of $10^{-6}$ mol/l, preferably less than $10^{-7}$ mol/l, most preferred $10^{-8}$ mol/l, or less.

Antibodies used according to the invention for the passive immunotherapy may be derived from a non-human species, such as a murine antibody. Yet it is expected that a recombinant, chimeric, as well as a humanized antibody combined with murine and human components, or a human antibody will be particularly tolerable for the administration on humans.

For the active immunization of cancer patients, components a) and/or b) are formulated in immunogenic form, or as vaccines, respectively. Preferred are pharmaceutical preparations which contain a pharmaceutically acceptable carrier. The latter comprises, e.g., auxiliary substances, buffers, salts, preserving agents. The pharmaceutical preparations may, e.g., be used for the prophylaxis and therapy of cancer-associated conditions, such as metastasis formation, in cancer patients. In doing so, antigen-presenting cells are specifically modulated in vivo or also ex vivo so as to generate the immune response against the TAAs.

For the active immunization with the specific antigens or the antigen combination, respectively, of the kit usually a vaccine formulation is used which contains the immunogen - be it a natural TAA or its epitope, mimic or neoepitope mimic, or an immunogenic antibody - mostly only at low concentrations, e.g. in an immunogenic amount ranging from 0.01 μg to 10 mg. Depending on the immunogenicity of the vaccination antigen which is, e.g., determined by sequences of a foreign species or by derivatization, or also depending on the auxiliary substances or adjuvants, respectively, used, the suitable immunogenic dose is chosen e.g. in the range of from 0.01 μg to 750 μg, preferably 100 μg to 500 μg. A depot vaccine which is to be delivered to the organism over an extended period of time may, however, also contain much higher amounts of vaccination antigen, e.g. at least 1 mg to more than 10 mg.

The concentration will depend on the amount of liquid or suspended vaccine administered. A vaccine usually is provided in ready-to-use syringes or ampoules having a volume ranging from 0.01 to 1 ml, preferably 0.1 to 0.75 ml.

The vaccination antigen of a component of the kit preferably is presented in a pharmaceutically acceptable carrier which is suitable for subcutaneous, intramuscular and also intradermal or transdermal administration. A further mode of administration functions via the mucosal pathway, e.g. vaccination by nasal or peroral administration. If solid substances are employed as auxiliary agent for the vaccine formulation, e.g. an adsorbate, or a suspended mixture, respectively, of the vaccine antigen with the auxiliary agent will be administered. In special embodiments, the vaccine is presented as a solution or a liquid vaccine in an aqueous solvent.

Preferably, vaccination units of a tumor vaccine are already provided in a suitable ready-to-use syringe or ampoule. A stable formulation of the vaccine may advantageously be put on the market in a ready to use form. Although a content of preserving agents, such as thimerosal or other preserving agents with an improved tolerability, is not necessarily required, yet it may be provided in the formulation for a longer stability at storage temperatures of from refrigerating temperatures up to room temperature. The vaccine however, may also be provided in frozen or lyophilized form and may be thawed or reconstituted, respectively, upon demand.

It has proved suitable to increase the immunogenicity of an antibody by employing adjuvants. For this purpose, solid substances or liquid vaccine adjuvants are used, e.g. aluminum hydroxide (Alu-Gel) or aluminum phosphate, growth factors, lymphokines, cytokines, such as IL-2, IL-12, GM-CSF, gamma interferon, or complement factors, such as C3d, further liposome preparations, or also formulations with additional antigens against which the immune system has already generated a strong immune response, such as tetanus toxoid, bacterial toxins, such as Pseudomonas exotoxins, and derivatives of lipid A and lipopolysaccharide.

Epitopes of the suitable target antigens imitate or comprise primarily domains of a natural, homologous or derivatized TAA. These are comparable to the TAA at least by their primary structure and, possibly, their secondary structure. The epitopes may, however, also be completely different in this respect from the TAAs and may imitate components of a TAA, primarily proteinaceous or carbohydrate antigens, respectively, simply by the similarity of spacial (tertiary) structures. Simply the tertiary structure of a molecule may, thus, form a mimic which triggers the immune response against a certain TAA.

At least two equal or different epitopes of an adhesion protein, e.g. of a homophilic cellular membrane protein, such as EpCAM, are provided, or imitated, respectively, on the antigen of component a). Thus, by the active immunization, a plurality of antibodies with a specificity for the same molecule, yet with different EpCAM binding sites may be generated. Likewise, also an epitope of an antigen of component b) can be conjugated to one or several of the epitopes of the adhesion protein. An accordingly formed "combination antigen" thus comprises at least one epitope, fragment or molecule of a cellular adhesion protein, such as EpCAM, and an epitope, fragment or molecule of an aberrant glycosylation, such as Lewis Y, on one single carrier. Such a combination antigen can imitate the cellular tumor antigens particularly well, and accordingly causes the desired immune response against the epithelial tumor cells.

For the vaccine formulation, also further known methods for conjugating or denaturing vaccine components may be used so as to further enhance the immunogenicity of the active substance. In addition, other substances, such as peptides, glycopeptides, carbohydrates, lipids or nucleic acids, yet also ionic groups, such as phosphate groups, or carrier molecules, such as polyethylene glycol or KLH, can covalently be bound to inventively used vaccination antigens. These side groups may possibly themselves represent epitopes of a tumor-associated antigen as defined by the present invention. One example of a conjugated vaccination antigen is the immunogenic antibody described in Austrian application A 744/2002 which is glycosylated with a Lewis Y antigenic structure.

In a combination antigen, the various epitope structures may be interconnected via a coupler. This coupler preferably is a short bifunctional molecule, such as, e.g., N-hydroxysuccinimide. Likewise, the coupler may also be realized by a chemical compound larger than a simple coupler molecule. It is always a prerequisite that this coupler will not have a negative influence on the immunogenic properties of the conjugate, i.e. that it by itself does not trigger any substantial immunogenicity. A coupler may also be produced by the chemical conversion of part of the vaccine antigen, or of the structure to be conjugated, respectively, practically "in situ". This coupler produced at the epitope structure itself may then be directly conjugated to the respective other binding partner (e.g. via the amine group of the lysine, via the OH groups, sulfur groups, etc.).

A vaccination antigen is also provided in the form of its corresponding nucleic acid. The nucleic acid encodes for a corresponding epitope and may optionally be directly inoculated as a "naked" nucleic acid so as to induce an immune response against the gene product in vivo.

Special embodiments of the vaccine contain, in particular, anti-idiotypic antibodies as vaccination antigens, i.e. ab2 which are directed against the idiotype of a TAA-specific antibody (ab1). Anti-idiotypic antibodies provoke in vivo the formation of ab3 antibodies which in turn also recognize the TAA of a tumor cell, bind thereto and lyse it accordingly. By way of example, an anti-idiotypic antibody against glycan-specific antibodies is used, such as an anti-idiotypic antibody which recognizes the idiotype of an anti-Lewis Y antibody, e.g. as described in EP 0 644 947.

These antibodies used for the active vaccination are administered in small amounts only. Thus, e.g., no special side effects are expected, even if the antibody has been derived from a non-human species, such as, e.g., a murine antibody. It is, however, assumed that a recombinant, chimeric antibody as well as a humanized antibody combined with murine and human components, or a human antibody is particularly well tolerable for the administration to humans. On the other hand, a murine portion in an inventive antibody on account of its foreignness may additionally provoke the immune response in humans.

The combination of the active and/or passive immunotherapy with adjuvant treatment methods known per se is very usual. Among them are means of radiotherapy or chemotherapy, such as the monotherapy or polytherapy. Because of the different mechanisms of action, the immunotherapy preferably is combined with the polychemotherapy. The combination of the active immunotherapy of a cancer patient with a chemotherapy has been described e.g. in Austrian application A 774/2002.

Agents preferably used for chemotherapy are alkylating pharmaceutical preparations. Thus, e.g., agents containing taxane, anthracyclines or platinum are preferred. All the conventional preparations which are employed for the various cancer treatments can be further combined. The chemotherapeutic agents usually are administered intravenously or perorally.

The treatment according to the invention comprises both prophylactic and therapeutic measures. The treatment is not only intended for the field of human medicine, but can also be used for the treatment and/or for diagnosing forms of cancer of various mammals. Patients with primary tumors can be treated just as patients with secondary tumors. The cancer treatment is particularly targeted to the treatment of the "minimal residual disease".

A possible objective of treatment is the effective binding and reduction of tumor cells so as to prevent their dissemination as far as possible. Simultaneously, also particularly the disseminated tumor cells are attacked. The tumor cells or micrometastases detectable in blood, bone marrow or organs are prophylactically prevented with the kit according to the invention, and significantly reduced therapeutically. The formation of metastases is to be retarded in that their growth is at least slowed down. Thus, by the immunotherapy according to the invention, the relapse-free life span and, thus, also the total time of survival of the patients can be extended. An indicator for the success of the treatment is the significant reduction of tumor cells in blood, serum or bone marrow.

The inventive kit of the antibodies also serves for the immunologic determination of a cancer disease. Since the said target antigens and the combination of the components a) and b) have proven particularly telling for the further course of the disease and the survival time, the active substances can be used as diagnostic means. In this manner, an immunologically active "panel" is provided which serves to select suitable diagnostic antibodies or is directly suitable as an immune reagent for determining tumor cells of solid tumors, metastases, micrometastases or disseminated tumor cells.

For the diagnostic determination, samples of tumor tissue or body fluids, such as blood or bone marrow, are taken from tumor patients. These samples then are admixed with the immunoreagents according to components a) and b) of the kit. A possible immune reaction is an indicator for the malignancy of the disease, and for the course of the disease, respectively, and a certain prognosis.

Small tumors or a sample of tumor tissue are usually taken by biopsy. Samples of tumor tissue are also obtained by the partial or complete resection of solid tumors. On account of the tumor tissue taken, a pathologist will then diagnose whether the tissue is from a benign tumor or from a malignant tumor. A diagnosis can be supplemented by the inventive method for immuno-histochemistry, or it may be offered as a standardized alternative.

The method of immunologically determining a cancer disease with at least one selection of immunoreagents according to components a) and b) can be further employed for examining samples of peripheral blood, bone marrow or fractions thereof. The advantageous use of a set according to the invention relates to the monitoring of cancer patients during a therapy. In particular, immunoreagents are used for determining an antibody titer against the antigens of components a) and b). Appropriate immunoreagents are, however, also used for the qualitative or even better, the quantitative determination of disseminated tumor cells from body fluids of a cancer patient during a therapy. The reduction of the disseminated tumor cells is an indication of the effectiveness of the therapy.

Monitoring during a cancer treatment preferably uses an inventive combination of immunoreagents, not only for determining a corresponding antibody titer in the patients' blood, but also for determining the effect on the tumor cells of solid tumors or on disseminated tumor cells.

Blood or serum samples from a cancer patient can be qualitatively and/or quantitatively examined for immune complexes with a diagnostic agent according to the invention. The analysis methods as such are common analysis methods with fractionation and enrichment of the immune complexes and/or immune reaction with a specific antibody, such as an antibody directed against the Fc portion of an antibody used for treatment. If the used antibody has murine structures, the latter can also be bound with an anti-murine antibody as in a capture step.

An immune reaction with the immunoreagents used according to the invention can be detected by an appropriate labelling of one of the binding partners from the immune complex. Usually an immunoreagent is provided with a labelling. A further preferred variant of the diagnostic agent additionally comprises a reagent which reacts with the immune complex possibly formed. This reaction can then be made visible by means of an appropriate labelling. The means for labelling immunoreagents or of reagents against the immunocomplexes are known to the person skilled in the art. Among them are fluorescent, chromogenic agents, radiolabels or enzyme labels.

Reagents for determining the components a) and/or b) or for determining their reaction products, respectively, or immune complexes advantageously are immobilized on a carrier. Suitable carriers are, e.g., prepared microtiter plates, yet also carriers suitable for immunoaffinity chromatography. The immune reagents advantageously are directly bound to column materials and bind the relevant antigens or antibodies from body fluids for determination thereof.

A preferred material for the diagnostic agent is, e.g., immobilized EpCAM, a recombinant EpCAM being preferred. This agent will then be combined with a further immobilized anti-idiotypic antibody having a specificity for the idiotype of a Lewis Y antibody or with an immobilized Lewis Y antigen. The preferred combination is the serial or parallel purification of immunoreactants from a body fluid, e.g. serum, so as to quantitate them.

Fig. 1 shows the co-expression of EpCAM and Lewis Y antigens on tumor tissue samples of female breast cancer patients, as well as their correlation with the survival time.

Fig. 2 shows Le-Y expression of different tumour cell lines, FACS results. Le-Y was detected with IGN311 and visualized using a FITC-conjugated anti-human antibody.

Fig. 3 shows the Her2-neu expression pattern of the same cell lines also analyzed by FACS using Herceptin® as detection antibody and the same secondary antibody system. The major difference to the previous analyzed Le-Y expression pattern was that WM9, Kato III and SKBR5 were Her2-neu negatives. Only SKBR3 expressed this membrane-protein. Therefore, only SKBR3 could be used despite its lower Le-Y expression density for ADCC experiments.

Fig. 4 shows the results of the Antibody Dependent Cellular Cytoxicity assay: Lysis of SKBR3 cells was mediated by effecter cells of a healthy donor in combination with IGN311, Herceptin® and combinations of constant IGN311 concentrations (40, 100 and 800 ng/ml) with Herceptin®.

In the following, the immunohistochemical determination of relevant antigens on tissue samples of solid tumors will be described, as well as diagnostic methods for the differentiated monitoring of immunospecific tumor markers. The following examples shall explain the present invention in more detail without, however, restricting it.

1. Immunohistochemical examination of microarrays

Tissue samples from solid tumors were prepared according to WO 0142796 as microarrays which were examined immunohistochemically by means of different antibody preparations. The determined antigen structures of the microarrays were compared with the history of the donors of the tissue samples.

As antibody preparations, the following were used:

ESA (Novocastra) mouse monoclonal anti-EpCAM antibody;
BR55-2 (EP 0 285 059, ATCC HB 9324) mouse monoclonal anti-Lewis Y antibody.

The immune reaction was determined by staining with a standard peroxidase-conjugated avidin-biotin system (Vector ABC-kit, vector, PK-6100) with diamino-benzidase as chromogen (DAB, Bio Genex, HK-130-5K).

The degree of staining was evaluated according to a score (0, 1+, 2+, 3+). In addition, the portion of positive cells was estimated. The tumor samples were classified according to staining and to the portion of the stained cells as follows:

Negative: no staining
weak: 1+ <70%, 2+ <20%
moderate: 1+ ≥70%, 2+ ≥20% and <80%, 3+ <30%
strong: 2+ ≥80%, 3+ ≥30%

A. Determination of the EpCAM expression on tissue samples of breast cancer

The reaction of the microarrays with ESA was more pronounced in the advanced state of the disease and with the degree of node formation. A missing staining with ESA correlated with a favorable prognosis. It was found that ESA preferably binds to samples of neoplastic breast diseases and tumors with an unfavorable prognosis. ESA, however, binds only weakly to normal breast tissue or to non-malignant breast diseases.

Table 1: ESA immune reaction and tumor stage

| Tumor stage | N | ESA Immune Staining | | | |
| --- | --- | --- | --- | --- | --- |
| | | Neg % | weak % | moderate % | strong % |
| pT1 | 631 | 54.04 | 21.24% | 14.58% | 10.14% |
| pT2 | 856 | 43.57% | 26.64% | 14.37% | 15.42% |
| pT3 | 106 | 42.45% | 24.53% | 16.98% | 16.04% |
| pT4 | 212 | 47.64% | 22.64% | 15.09% | 14.62% |

p = 0.0078
N - number of microarrays examined

Table 2: ESA immune reaction and tumor degree

| Tumor degree | N | ESA Immune Staining | | | |
|---|---|---|---|---|---|
| | | Neg % | weak % | moderate % | strong % |
| G1 | 429 | 64.57% | 20.51% | 8.62% | 6.29% |
| G2 | 693 | 52.67% | 25.11% | 13.85% | 8.37% |
| G3 | 572 | 26.40% | 26.75% | 20.63% | 26.22% |
| P <0.0001 | | | | | |

Table 3: ESA immune reaction and node status

| Node status | N | ESA Immune Staining | | | |
|---|---|---|---|---|---|
| | | Neg % | weak % | moderate % | strong % |
| pN0 | 744 | 50.67% | 20.97% | 13.98% | 14.38% |
| pN1 | 654 | 47.09% | 25.38% | 15.44% | 12.08% |
| pN2 | 105 | 33.33% | 27.62% | 18.10% | 20.95% |
| P = 0.0115 | | | | | |

B. Determination of the Lewis Y expression on tissue samples of breast cancer

Lewis Y is expressed on different samples of normal tissue and on tissue from neoplastic breast diseases. The reaction of the microarrays with BR55-2 was more pronounced with an advanced degree of tumor, yet did not correlate with the node status and tumor stage.
(Sauter et al., Abstract 107052, Meeting report, Eurocancer 2003)

Table 4: BR55-2 immune reaction and tumor stage

| Tumor stage | N | Lewis Y Immune Staining | | | |
|---|---|---|---|---|---|
| | | Neg % | weak % | moderate % | strong % |
| PT1 | 597 | 20.44% | 30.15% | 32.66% | 16.75% |
| PT2 | 834 | 20.62% | 31.65% | 34.77% | 12.95% |
| PT3 | 95 | 12.63% | 35.79% | 41.05% | 10.53% |
| PT4 | 206 | 20.39% | 34.95% | 31.07% | 13.59% |
| p = 0.2486 | | | | | |

Table 5: BR55-2 immune reaction and tumor degree Lewis Y Immune Staining

| Tumor degree | N | Neg % | weak % | moderate % | strong % |
|---|---|---|---|---|---|
| G1 | 412 | 24.76% | 24.03% | 34.71% | 16.50% |
| G2 | 652 | 19.94% | 31.90% | 36.50% | 11.66% |
| G3 | 552 | 16.85% | 38.22% | 29.53% | 15.40% |
| P <0.0001 | | | | | |

Table 6: BR55-2 immune reaction and node status

| Node status | N | Lewis Y Immune Staining | | | |
|---|---|---|---|---|---|
| | | Neg % | weak % | moderate % | strong % |
| PN0 | 713 | 21.04% | 29.31% | 34.92% | 14.73% |
| PN1 | 629 | 20.67% | 33.39% | 31.64% | 14.31% |
| PN2 | 99 | 13.13% | 39.39% | 34.34% | 13.13% |
| p = 0.2621 | | | | | |

C. Determination of the EpCAM and Lewis Y co-expression on tissue samples of breast cancer

The co-expression of EpCAM and Lewis Y antigen on breast cancer tissue is found in many cases. The probability for the EpCAM specific immune staining increases with the color intensity of the Lewis Y immune staining. The co-expression is primarily frequently found in high-degree tumors, yet it is hardly associated with the tumor stage or node stage.

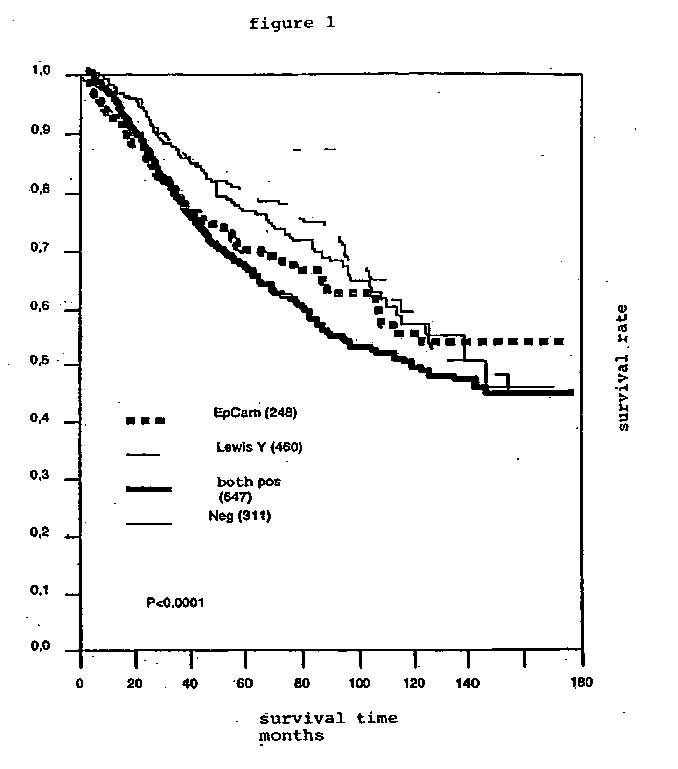

The prognosis for the survival time of the cancer patients is clearly deteriorated if both antigens are expressed (p<0.0001), cf. Fig. 1 in this context.

Table 7: ESA and BR55-2 immune reaction and tumor stage

| Tumor stage | N | ESA Immune Staining | | | |
| | | Neg % | Lewis Y % | EpCam % | both pos % |
| --- | --- | --- | --- | --- | --- |
| PT1 | 568 | 41.73% | 32.92% | 10.92% | 14.44% |
| PT2 | 799 | 39.55% | 29.66% | 15.64% | 15.14% |
| PT3 | 93 | 38.71% | 24.73% | 16.13% | 20.43% |
| PT4 | 202 | 36.63% | 33.17% | 14.85% | 15.35% |
| p = 0.2482 | | | | | |

Table 8: ESA and BR55-2 immune reaction and tumor degree

| Tumor degree | N | ESA Immune Staining | | | |
| | | Neg % | Lewis Y % | EpCam % | both pos % |
| --- | --- | --- | --- | --- | --- |
| G1 | 393 | 50.38% | 34.10% | 8.91% | 6.62% |
| G2 | 627 | 44.66% | 32.06% | 11.32% | 11.96% |
| G3 | 536 | 25.19% | 27.24% | 21.46% | 26.12% |
| p<0.0001 | | | | | |

Table 9: ESA and BR55-2 immune reaction and node status

| Node status | N | ESA Immune Staining | | | |
| | | Neg% | Lewis Y % | EpCam % | both pos % |
| --- | --- | --- | --- | --- | --- |
| PN0 | 679 | 42.56% | 27.98% | 13.70% | 15.76% |
| PN1 | 605 | 38.84% | 32.56% | 13.06% | 15.54% |
| PN2 | 98 | 30.61% | 30.61% | 16.33% | 22.45% |
| P = 0.1746 | | | | | |

2. Monitoring the immune response to an active immunization

In a study regarding the active immunization of rhesus monkeys with a vaccine based on a He2-Lewis Y neoglycoprotein (HE2LeY), prepared according to Austrian application A744/2002, the success of immunization was determined by way of the specific immune response.

A diagnostic agent was used so as to determine the titer of anti-EpCAM antibodies and anti-Lewis Y antibodies via the sequential immune affinity chromatography. The affinity chromatography was effected with the assistance of an FPLC system. 1 ml of serum was diluted 1:10 in phosphate-buffered saline and applied to the first column. The bound fraction was recovered by elution with a glycine buffer or with ammonium, and optionally applied to a second column. The immunoglobulin content of the EpCAM and Lewis Y fractions was quantitated.

The following column materials were used for the diagnostic determination:

1. EpCAM-Sepharose: recombinant EpCAM bound to a CH-Sepharose 4B column
2. SynsorbY: Lewis Y bound to a chromo sorb matrix (of Synsorb)
3. He2LeY Sepharose: He2LeY bound to a CH-Sepharose 4B column

Columns 1 and 2 were used both as sole diagnostic agents as well as in series.

3. Determination of disseminated tumor cells from peripheral blood

Disseminated tumor cells were determined from blood samples of tumor patients in the following manner:

A. Tumor cell enrichment:

25 ml of peripheral blood were centrifuged at 1600 x g for 20 min at 4°C in an OncoQuick® tube (Greiner bio-one, Altmünster, Austria). The tumor cell-containing phase was transferred into a further centrifuge tube and a cell pellet was recovered by centrifuging. This pellet was resuspended. The cellular portion of the suspension was centrifuged to a slide for microscopic examination. The slide was stored at -20°C until evaluation.

B. Tumor cell determination

A soltuion with fluorescence-labelled specific antibodies was applied to the slide with the isolated and enriched tumor cells. After a period of incubation of 30 min, the tumor cells labelled by the antibody bond were visualized under the fluorescence microscope (Axioplan Zeiss, Jena, Germany) and counted. The content of tumor cells in the blood was then calculated according to the factor of enrichment. The method was validated by standard tumor cell suspensions.
IGN311, a humanized monoclonal anti-Lewis Y antibody, prepared according to EP 528 767, and HEA-FITC (Anti-EpCAM, Miltenyi, clone HEA125) both conjugated with fluorescent proteins, are utilized as labelled, specific antibodies.
The effectiveness of the immunotherapeutic treatment of cancer patients is proven by the detection of the tumor cells in peripheral blood. The reaction of detected tumor cells with one of the two antibodies or with both antibodies is differentiatedly evaluated within the scope of monitoring.

4. Selection of antibodies with a specificity for a neo-epitope

BALB/c mice are immunized at first intraperitoneally and 4 weeks later intravenously with cells of the SKBR3 breast cancer cell line. Spleen cells are fused with suitable melanoma cells, and hybridomas are prepared. The monoclonal antibodies obtainable therefrom are examined for their specificity with a differential screening method. The appropriate positive clones are used for production of the neo-epitope specific monoclonal antibodies so as to produce antibody preparations for the immunotherapeutic treatment of tumor patients.
Differential screening is effected consecutively with the following antigens:

A. Lewis Y-Her-2/neu receptor (SKBR3 cell membrane extract on Western Blot)
B. Lewis Y (synthetic, Synthesom)
C. Her-2/neu receptor, recombinant

The neo-epitope antibodies bind only to antigen A, yet not to the antigens B or C.

Example 2: Combinatorial targeting of tumor cell related protein and oligo-saccharide antigens

Two tumor associated antigens, the Le-Y glycosylation pattern and the membrane protein Her2-neu were chosen as simultaneous targets in ADCC experiments. SKBR3 cell lines was selected between different other tumor cell lines over-expressing both antigens at highest densities. Serial dilutions of both antibodies were incubated with target cell line and PBMC's from a healthy donor. ADCC was analyzed using a $^{51}$Cr release assay for both antibodies, half time lysis were 1.1 $\mu$g/ml and 0.4 ng/ml for IGN311 and Herceptin® respectively. 40, 100 and 800 ng/ml IG-N311 were added as constant concentration to the serial Herceptin® dilution. Calculated Hill-slopes of lysis curves near their inflection point increased from nearly 1.0 for Herceptin® (same value for IGN311) to values of 2.6, 3.0 and 3.6 with raised concentrations of simultaneously supplemented IGN311. Since these values exceed the effect of summarized individual effects, we could observe a synergistic effect by simultaneously targeting both tumor associated antigen structures Le-Y and Her2-neu.
A purpose of a combined simultaneous targeting of different tumor cell related antigens is an enhancement of the involved individual mechanisms leading finally to more effective tumor cell lysis. Our chosen target-structures in this example were the membrane anchored protein Her2-neu and the membranous Le-Y glycosylation pattern. Both antigenes are epithelial tumor-cell related and over-expressed on 70% of all epithelial tumors.

Material and Methods

IGN311 is a humanized anti-Le-Y antibody. Herceptin® is a commercial available antibody. Cell lines Kato III, WM9 and SKBR3 were purchased from ATCC.

ADCC experiments were carried out as follows: $10^7$ SKBR 3 cells were seeded in a 75 cm$^2$ flask and grown for 4 days. 110 ml of blood/plate were collected in Vacutainer CPT (Cell preparation tubes) from a healthy donor. PBMC's were isolated and washed twice with 0.1 % glucose in PBS complete, once spinning at 400 g, second time spinning at 75 g. $7.5 \times 10^5$ isolated cells were seeded per well. Cells were incubated overnight. Target cells were scraped the out of their flask and monodispersed in media. $25 \times 10^3$ cells were seeded per well. They are resuspended in small amount of media (850 μl) and incubated with $^{51}CrO_4^{2-}$ for one hour. Cells were washed twice afterwards with media and resuspended. Cell concentration was adjusted to [number of PBMC's per well] divided by 30 in 100 μl to reach an effecter to target ratio of 30 to 1. Serial dilutions of the antibody(ies) to analyse were prepared in media. In an ELISA-plate holding the PBMC's, 50 μl of antibody dilution (or media) were added, then 100 μl of cell suspension was supplemented. Suspension is incubated for 18 hours in a $CO_2$ incubator. Supernatant is harvested and the intensity of y - radiation is counted. Two controls are prepared on the plate:

Maximum lyses, receiving (in this order) 50 μl of media, 100 μl of cell suspension and 100 μl of SDS - solution; spontaneous release, receiving 150 μl of media and 100 μl of cell suspension. Percent lyses in sample is calculated with Formula 1:

Calculation of lyses in ADCC experiments:

$$\% \text{ lyses} = ((CPM_{(Sample)} - CPM_{(Spontaneous\ Release)})/ (CPM_{(Maximum\ Lyses)} - CPM_{(Spontaneous\ Release)})) \times 100$$

Results

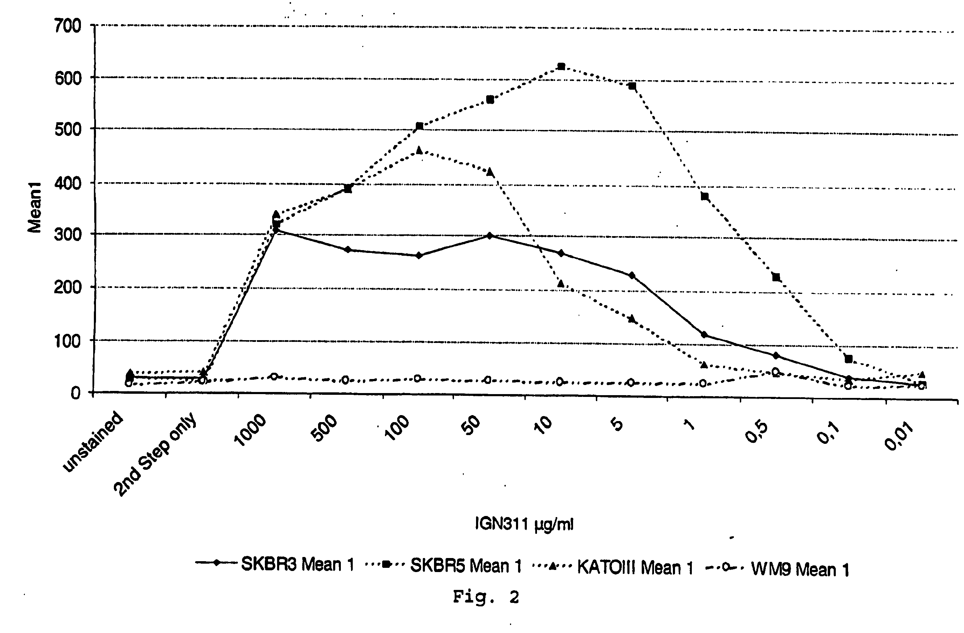

One important requirement for the used cell line was the simultaneous over-expression of both antigens Le-Y and Her2-neu. Different cell lines were screened for these parameters in FACS experiments. Kato III, SKBR3 and SKBR5 were analyzed. Figure 2 shows the FACS results concerning the Le-Y expression pattern. Humanized anti Le-Y antibody IGN311 was used to detect Le-Y glycosylation pattern. All three epithelial cancer lines were compared to the melanoma cell line WM9. All three cell lines expressed membranous Le-Y.

The results are shown in figure 2 shows the Le-Y expression of different tumour cell lines, FACS results. Le-Y was detected with IGN311 and visualized using a FITC-conjugated anti-human antibody.

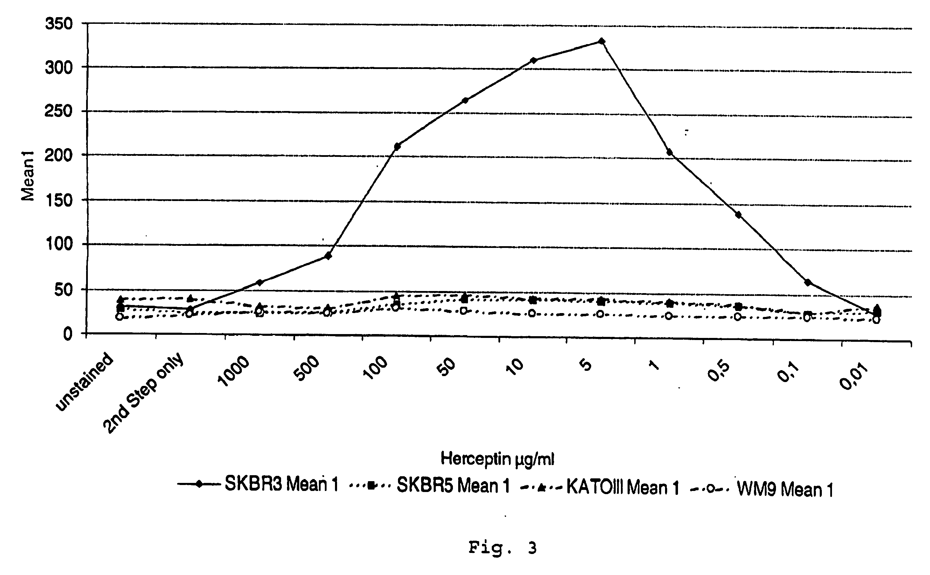

Figure 3 shows the Her2-neu expression pattern of the same cell lines also analyzed by FACS using Herceptin® as detection antibody and the same secondary antibody system. The major difference to the previous analyzed Le-Y expression pattern was that WM9, Kato III and SKBR5 were Her2-neu negatives. Only SKBR3 expressed this membrane-protein. Therefore, only SKBR3 could be used despite its lower Le-Y expression density for ADCC experiments.

The results are shown in figure 3: HER2-neu expression pattern of different tumour cell lines, FACS results. Her2-neu was detected with Herceptin® and visualized using a FITC-conjugated anti-human antibody.

Antibody Dependent Cellular Cytotoxicity chromium release experiment, were carried out using SKBR3 target cells. They were incubated with $^{51}Cr$ for one hour. Then, effecter cells from healthy donors (PBMC preparation) were supplemented with serial dilutions of IGN311, Herceptin® and combinations of both antibodies. In the case of Herceptin®, a serial dilution from 0.4 pg/ml to 40 ng/ml was analyzed, in the case of IGN311 from 2 ng/ml to 10 μg/ml. The combination of Herceptin® and IGN311 consisted of the identical serial Herceptin® dilution supplemented with three constant IGN311 concentration of 40, 100 and 800 ng/ml.

SKBR3 cells were incubated over night (18 hours) with prepared effector suspensions (effecter cells with antibody solution).

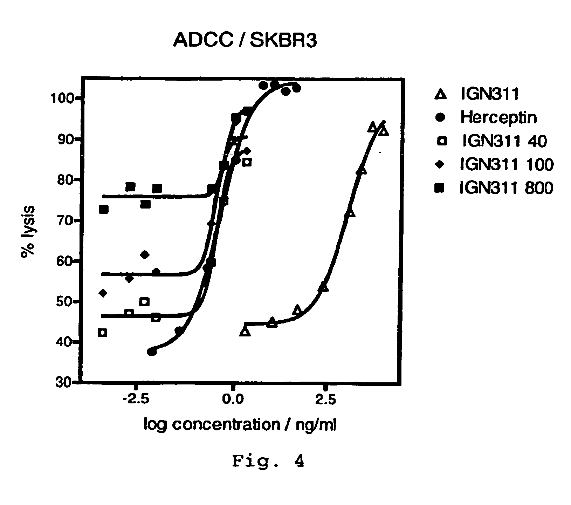

Triplicate measurements were performed. Chromium release, a function of cell lysis, was measured in supernatants using a gamma-radiation counter. Lysis potential was calculated as described in Methods section. Data were fitted using a sigmoidal curve fit model (GraphPad Prism software, Table 1) and results are displayed graphically in Figure 4.

Table 1: ADCC results: 4 Parameter sigmoidal curve fit results evaluated by GraphPad Prism

|  | IGN311 | Herceptin® | IGN311 40 | IGN311 100 | IGN311 800 |
|---|---|---|---|---|---|
| BOTTOM | 44,59 | 37,53 | 46,49 | 56,81 | 76,05 |

(continued)

|  | IGN311 | Herceptin® | IGN311 40 | IGN311 100 | IGN311 800 |
|---|---|---|---|---|---|
| TOP | 99,3 | 104,5 | 87,88 | 90,9 | 97,65 |
| LOGEC50 | 3,043 | -0,3852 | -0,4668 | -0,515 | -0,2398 |
| **HILLSLOPE** | **1,071** | **1,116** | **2,642** | **2,998** | **3,592** |
| EC50 | 1103 | 0,4119 | 0,3413 | 0,3055 | 0,5758 |

Figure 4 shows the results of the Antibody Dependent Cellular Cytoxicity assay: Lysis of SKBR3 cells was mediated by effecter cells of a healthy donor in combination with IGN311, Herceptin® and combinations of constant IGN311 concentrations (40, 100 and 800 ng/ml) with Herceptin®.

All curves showed in their maximal concentration range a lysis potential of 89 to 104%. In the lower concentration range, bottom values of 38 and 45% were obtained for Herceptin® and IGN311. These values also corresponded to the ones measured for spontaneous lysis phenomena in this experimental setup (data not shown). IGN311 showed a half-time lysis by 1.1 $\mu$g/ml while the one of Herceptin® was in the range of 0.4 ng/ml. For the combinatorial setup, constant IGN311 concentrations(40, 100 and 800 ng/ml) were chosen in the dynamic range of the curve.

The Hillslope value (Table 1) describes the slope of the fitted sigmoidal curve near its inflection-point. This parameter is essential for comparison of effects due to combination of both antibodies. IGN311 and Herceptin®, both supplemented alone to the effecter cells, showed a hillslope nearly equal to 1.0.

If both antibodies would react independently from each other, the combination should not affect the resulting slope near the inflection point of lysis curves although a shift to higher lysis potential should be visible. If there would be an inhibition of both antibodies, the resulting slope near the inflection point of lysis curves would be lowered.

As result of a synergistic effect, hill-slope would increase. This is the observe effect in our assay. The combination of Herceptin® with 40 ng/ml IGN311 resulted in a shift of the resulting slope near the inflection point of lysis curves from 1.0 to 2.6. The supplementation with 100 ng/ml and 800 ng/ml resulted even in slopes increased to values of 3.0 and 3.6 respectively. A synergistic effect may therefore have occurred.

Combination of IGN311 and Herceptin® leads to an enhanced lysis potential, greater than the effect that would have been observed by the sum of the individual components. This or an equivalent combination targeting both membrane anchored (glyco-)proteins and glycostructures over-expressed in cancer cells may play an important role in passive immunotherapy of cancer.

Example 3: Selection of an EpCAM-LewisY neoepitope specific antibody

1. Immunization with tumor cells:

a)mice are immunized with EpCAM- and LewisY-positive Tumor tissue according to standard methods. After preparation of hybridomas the hybridoma clones are selected for neoepitope specificity by differential screening with assays detecting the binding of the hybridoma derived monoclonal antibodies to

A: EpCAM-and LewisY positive cells

B: normal epithelial cells (EpCAM positive, Lewis Y-negative cells)

C: EpCAM-negative/Lewis Y-positive cells

Binding assays can be performed by eg. FACS analysis or immuno-western blotting with electrophoretically separated cell extracts.

Antibodies specific for the neoepitopes are binding in assay A, but not in assays B and C.

2. Immunization with recombinant EpCAM and sugar: Alternatively, purified or recombinant EpCAM can be used as well as synthetic Lewis Y can be used for immunization and for screening.

Alternatively, screening for other aberrant glycosylation forms such as sialyl-Tn, etc. can be screened for in assay C. In this case, Lewis Y glycosylation is replaced by the other sugar moiety.

3. Alternatively, the neoepitope-specific ligands can be selected by reacting libraries of ligands (such as scFv-, Fab-, antibody-libraries, peptide libraries, random libraries using other scaffolds than immunoglobulins).

4. Mimics for the neoepitope can be selected by using a neoepitope-specific ligand as antigen. It can be used as an immunogen or antigen to produce anti-idiotypic antibodies by standard techniques such as hybridoma technique or screening with specific binding-pair libraries (eg. antibody-, Fab-, scFv-libraries) or by screening (panning) with other random libraries (non-immunoglobulin scaffolds, for example "repeat proteins" such as ankyrin, leucine-rich or armadillo repeat protein, but also anticalins (Arne Skerra, in Recombinant antibodies, 29-30 April 2003, Meeting report; Andreas Plückthun, in Recombinant antibodies, 29-30 April 2003, Meeting report), peptides).

The selected mimics are binding to the neoepitope-specific ligand and can be displaced by natural, neoepitope containing structures (eg cells expressing the neoepitope).

## Claims

1. A kit for the combined use for the treatment of cancer patients, which set comprises the following components:

   a) an antibody directed against a tumor cell surface protein, which is Lewis Y glycosylated in cancer, selected from a receptor of the EGF-receptor family and EpCAM, and
   b) an antibody directed against the Lewis Y antigen,

   said antibodies a) and b) having antibody-dependent cellular cytotoxicity and bind to the tumour cells in the patient thereby forming immune complexes.

2. A kit according to claim 1, **characterized in that** the components a) and b) are contained in one pharmaceutical preparation each or in a single pharmaceutical preparation suitable for immunotherapy.

3. A kit according to claim 2, **characterized in that** the pharmaceutical preparation is formulated as an intravenously tolerable product.

4. A kit according to any one of claims 1 to 3, **characterized in that** the antibody of component a) binds to an epitope of the Her-2/neu receptor.

5. The combined use of

   a) an anti-EpCAM antibody or anti-Her2-neu antibody; and
   b) an anti-Lewis Y antibody

   for the determination of tumor cells of a solid tumor or disseminated tumor cells of a cancer disease by determining an immune reaction with said antibodies in a sample of a tumor tissue or body fluids in the prognosis of the disease.

6. The use according to claim 5, **characterized in that** the sample is of peripheral blood or bone marrow.

## Patentansprüche

1. Kit für die kombinierte Verwendung zur Behandlung von Krebspatienten, welcher Satz die folgenden Komponenten umfasst:

   a) einen Antikörper, der gegen ein Tumorzelloberflächenprotein gerichtet ist, welches bei Krebs Lewis Y-glykosyliert ist, ausgewählt aus einem Rezeptor der EGF-Rezeptorfamilie und EpCAM, und
   b) einen Antikörper, der gegen das Lewis Y-Antigen gerichtet ist,

   wobei die Antikörper a) und b) eine Antikörper-abhängige zelluläre Zytotoxizität aufweisen und an die Tumorzellen in dem Patienten binden, wodurch sie Immunkomplexe bilden.

2. Kit gemäß Anspruch 1, **dadurch gekennzeichnet, dass** die Komponenten a) und b) jeweils in einer pharmazeutischen Präparation oder in einer einzigen pharmazeutischen Präparation, die jeweils für die Immuntherapie geeignet sind, enthalten sind.

**3.** Kit gemäß Anspruch 2, **dadurch gekennzeichnet, dass** die pharmazeutische Präparation als ein intravenös tolerierbares Produkt formuliert ist.

**4.** Kit gemäß einem der Ansprüche 1 bis 3, **dadurch gekennzeichnet, dass** der Antikörper der Komponente a) an ein Epitop des Her-2/neu-Rezeptors bindet.

**5.** Kombinierte Verwendung

a) eines Anti-EpCAM-Antikörpers oder Anti-Her2/neu-Antikörpers; und
b) eines Anti-Lewis Y-Antikörpers

zur Bestimmung von Tumorzellen eines festen Tumors oder von disseminierten Tumorzellen einer Krebserkrankung, indem eine Immunreaktion mit diesen Antikörpern in einer Probe von einem Tumorgewebe oder von Körperflüssigkeiten in der Prognose der Erkrankung bestimmt wird.

**6.** Verwendung gemäß Anspruch 5, **dadurch gekennzeichnet, dass** die Probe von peripherem Blut oder Knochenmark ist.

**Revendications**

**1.** Kit pour une utilisation combinée dans le traitement de patients cancéreux, lequel ensemble comprend les composants suivants :

a) un anticorps dirigé contre une protéine de surface de la cellule tumorale, qui est glycosylée par l'antigène Lewis Y dans le cancer, choisie parmi un récepteur de la famille des récepteurs de l'EGF et EpCAM, et
b) un anticorps dirigé contre l'antigène Lewis Y, lesdits anticorps a) et b) étant dotés d'une cytotoxicité cellulaire dépendante de l'anticorps et se liant aux cellules tumorales chez le patient, formant de cette façon des complexes immuns.

**2.** Kit selon la revendication 1, **caractérisé en ce que** les composants a) et b) sont contenus dans une préparation pharmaceutique chacun ou dans une seule préparation pharmaceutique appropriée pour une immunothérapie.

**3.** Kit selon la revendication 2, **caractérisé en ce que** la préparation pharmaceutique est formulée sous la forme d'un produit tolérable par voie intraveineuse.

**4.** Kit selon l'une quelconque des revendications 1 à 3, **caractérisé en ce que** l'anticorps du composant a) se lie à un épitope du récepteur Her-2/neu.

**5.** Utilisation combinée de

a) un anticorps anti-EpCAM ou un anticorps anti-Her2-neu ; et
b) un anticorps anti-Lewis Y

pour la détermination de cellules tumorales d'une tumeur solide ou de cellules tumorales disséminées d'une maladie cancéreuse par détermination d'une réaction immunitaire avec lesdits anticorps dans un échantillon d'un tissu tumoral ou de liquides corporels dans le pronostic de la maladie.

**6.** Utilisation selon la revendication 5, **caractérisée en ce que** l'échantillon est du sang périphérique ou de la moelle osseuse.

EP 1 528 936 B1

figure 1

figure 1

19

Fig. 2

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1140168 A
- EP 0528767 A
- AU 7442002 A
- WO 0135989 A
- WO 0006605 A
- WO 03003985 A
- EP 1140168 B1
- AU 9952002 A
- EP 0644947 A
- AU 7742002 A
- WO 0142796 A
- EP 0285059 A
- EP 528767 A

### Non-patent literature cited in the description

- *Immunology Today,* 2000, vol. 21, 403-410
- *Curr. Opin. Immunol.,* 1997, vol. 9, 717
- *Ann. Med.,* 1999, vol. 31, 66
- *Immunobiol.,* 1999, vol. 201, 1
- *Immunol. Today,* 2000, vol. 21, 403
- *Toxicon,* 1998, vol. 36, 823
- *Therapie,* 1994, vol. 49, 41
- *Clin. Infect. Dis.,* 1995, vol. 21, 150
- *Glycoconj. J.,* 1997, vol. 14, 569
- *Adv. Cancer Res.,* 1989, vol. 52, 257
- *Cancer Res.,* 1996, vol. 56, 5309
- *Annu. Rev. Biochem.,* 1988, vol. 57, 785
- *N. Engl. J. Med.,* 1992, vol. 327, 14
- *Elektrophoresis,* 1999, vol. 20, 362
- *Curr. Pharmaceutical Design,* 2000, vol. 6, 485
- *Neoplasma,* 1996, vol. 43, 285
- *Sem. Cancer Biol.,* 1995, vol. 6, 321
- **ALLERGOL.** *Immunopathol.,* 1997, vol. 25 (4), 176-81
- **FLIEGER D. et al.** *Cancer Immunology Immunotherapy,* 2000, vol. 49, 441-448
- **KIM S.K. et al.** *Vaccine,* 2000, vol. 19, 530-537
- **DI CARLO A. et al.** *Oncology Reports,* 2001, vol. 8, 387-392
- **GRATSA A. et al.** *Anticancer Research,* 1997, vol. 17, 4111-4117
- **NANASHIMA A. et al.** *Journal of Hepato-Biliary-Pancreatic Surgery,* 1999, vol. 6, 391-395
- **SANDERS D.S.A. et al.** *Molecular Pathology,* 1999, vol. 52, 174-178
- **LIVINGSTON et al.** *Journal of Clinical Oncology,* 1994, vol. 12 (5), 1036-1044
- **KIM et al.** *Vaccine,* 2000, vol. 18, 597-603
- **RAGUPAHTI et al.** *Proceedings of the annual meeting of the American Association for Cancer Research,* 2000, vol. 41 (874/875), 5557
- **ZHANG et al.** *Clin. Can. Res,* 1998, vol. 4 (2), 295-302
- **ARNE SKERRA.** *Recombinant antibodies,* 29 April 2003
- **ANDREAS PLÜCKTHUN.** *Recombinant antibodies,* 29 April 2003